(19) Europäisches Patentamt
European Patent Office
Office européen des brevets

(11) EP 3 656 372 A1

(12) **EUROPEAN PATENT APPLICATION**
published in accordance with Art. 153(4) EPC

(43) Date of publication:
**27.05.2020 Bulletin 2020/22**

(21) Application number: **18835504.4**

(22) Date of filing: **12.07.2018**

(51) Int Cl.:
***A61K 8/81*** *(2006.01)* ***A61Q 1/10*** *(2006.01)*
***A61Q 3/02*** *(2006.01)*

(86) International application number:
**PCT/JP2018/026358**

(87) International publication number:
**WO 2019/017275 (24.01.2019 Gazette 2019/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.07.2017 JP 2017138843**

(71) Applicant: **Japan Beauty Products Co., Ltd.**
**Tokyo 104-0061 (JP)**

(72) Inventors:
- **NAGAI Keiichi**
**Tokyo 104-0061 (JP)**
- **YOKOI Ayako**
**Tokyo 104-0061 (JP)**

(74) Representative: **Barker Brettell LLP**
**100 Hagley Road**
**Edgbaston**
**Birmingham B16 8QQ (GB)**

(54) **COSMETIC**

(57) A cosmetic is provided, including a component (R): synthetic rubber particles, and a component (P): a polymer having a repeating unit (u1) represented by General Formula (p1). In General Formula (p1), $R^{01}$, $R^{02}$ and $R^{03}$ each independently represents an alkyl group or a hydrogen atom; $n_p$ represents an integer of 1 to 4; $R^{10}$ represents a substituent; and $m_p$ represents an integer of 0 to $(n_p+1)$.

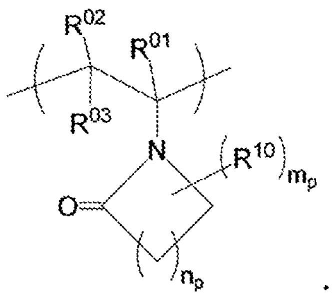

··· (p 1)

EP 3 656 372 A1

## Description

[Technical Field]

The present invention relates to a cosmetic.

Priority is claimed on Japanese Patent Application No. 2017-138843, filed July 18, 2017, the content of which is incorporated herein by reference.

[Background Art]

Various cosmetics have been marketed in the past due to the demand for beautiful eyes and fingertips.

In response to such demand, for example, a method of forming a double eyelid without depending on an orthopedic operation, a method of attaching false eyelashes to the outer corner and inner corner of the eyes, a method of applying a manicure preparation to the surface of the nails, and the like are used.

In the method of forming a double eyelid, a special treatment agent that is applied to the upper eyelid of a human is used to create a double fold state.

In the method of attaching false eyelashes from the outer corner to the inner corner of the eyes, in order to attach the false eyelashes at predetermined positions, a special adhesive is used.

In the method of applying a manicure preparation to the surface of the nails, in order to prevent chemicals from spreading outside of a desired area, a nail spreading prevention agent is applied to the fingertips.

In any of the above methods, it is important that a film formed on the skin using a coating agent be able to be easily peeled off from the skin.

For example, examples of methods of forming a double eyelid include a method of simply applying a treatment agent to the upper eyelid of a human and a method of forming a new crease part by pressing a treatment agent application part with a special member and maintaining this state.

In Patent Literature 1, regarding a treatment agent that is applied to the lower edge part of the upper eyelid and dried to form a double eyelid, a double eyelid forming treatment agent composed of 85 to 50 weight% of natural rubber latex and 15 to 50 weight% of a synthetic resin (an acrylic resin, a urethane resin, or a vinyl resin) emulsion is disclosed. Since a film formed using such a treatment agent contains a natural rubber latex, an adhesive force, elastic properties and ease of peeling off from the skin (easy peelability) (hereinafter collectively referred to as a "feeling of use") are improved.

[Citation List]

[Patent Literature]

[Patent Literature 1]
Japanese Unexamined Patent Application, First Publication No. H2-188512

[Summary of Invention]

[Technical Problem]

However, a treatment agent containing natural rubber latex as disclosed in Patent Literature 1 has problems such as change (discoloration, separation, etc.) in the appearance and generation of odor during storage over time.

The present invention has been made in view of the above circumstances, and an object of the present invention is to provide a cosmetic having both an excellent feeling of use (an adhesive force, a film elastic property, easy peelability, etc.) and stability over time.

[Solution to Problem]

Change in the appearance and generation of odor during storage over time can be reduced by simply replacing a natural rubber with a synthetic rubber. However, use of a synthetic rubber is inferior to use of a natural rubber in consideration of a feeling of use.

According to studies by the inventors, it has been found that, when a synthetic rubber is used in place of a natural rubber and a specific polymer having a lactam structure is combined therewith, the stability over time can be secured, and in addition, a feeling of use (an adhesive force, a film elastic property, easy peelability, etc.) equal to or better than that when a natural rubber is used can be exhibited and thereby the present invention has been completed.

That is, the cosmetic of the present invention includes a component (R): synthetic rubber particles, and a component (P): a polymer having a repeating unit (u1) represented by the following General Formula (p1):

[Chem. 1]

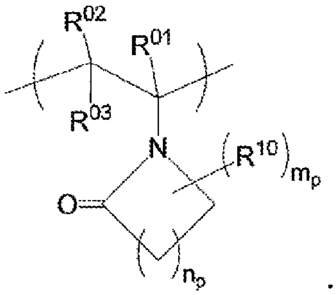

... (p 1)

wherein $R^{01}$, $R^{02}$ and $R^{03}$ each independently represents an alkyl group or a hydrogen atom; $n_p$ represents an integer of 1 to 4; $R^{10}$ represents a substituent; and $m_p$ represents an integer of 0 to $(n_p+1)$.

In the cosmetic of the present invention, the polymer for the component (P) may further include a repeating unit (u2) represented by the following General Formula (p2):

[Chem. 2]

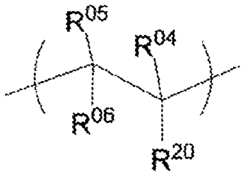

... (p 2)

wherein $R^{04}$, $R^{05}$ and $R^{06}$ each independently represents an alkyl group or a hydrogen atom; $R^{20}$ represents *-O-C(=O)-$R^{201}$, *-C(=O)-O-$R^{202}$, or *-C(=O)-NH-$R^{203}$; $R^{201}$, $R^{202}$ and $R^{203}$ each represents an organic group; and * indicates a bond at the $\alpha$-position carbon atom.

Preferably, the cosmetic of the present invention further includes an acrylic polymer.

The cosmetic of the present invention can be appropriately used as a double eyelid forming treatment agent, a false eyelash adhesive, a nail spreading prevention agent or a body paint.

[Advantageous Effects of Invention]

According to the present invention, it is possible to provide a cosmetic having both an excellent feeling of use (an adhesive force, a film elastic property, easy peelability, etc.) and stability over time.

[Description of Embodiments]

(Cosmetic)

Regarding one embodiment of a cosmetic according to the present invention, a liquid composition including a component (R): synthetic rubber particles, a component (P): a polymer having a repeating unit (u1)represented by General Formula (p1), and a solvent (hereinafter referred to as a "component (S)") may be exemplified.

"Cosmetic" in this specification refers to those defined in Article 2, Paragraph 3 of "the Law for Ensuring Quality, Efficacy, and Safety of Drugs and Medical Devices," that is, includes products that are used in order to clean, beautify, enhance the attractiveness or change the appearance of a human body, or keep the skin or hair healthy according to a method of rubbing or spraying it on the body, or other similar methods, and products that have a mild effect on the human body and products equivalent thereto.

Examples of products corresponding to the above products include those corresponding to quasi-drugs and

those corresponding to miscellaneous products.

For example, the cosmetic of the present embodiment can be appropriately used as a composition for a product that enables the eyes or fingertips to appear beautiful.

Examples of materials for the eyes include a double eyelid forming treatment agent and a false eyelash adhesive. Examples of materials for the fingertips include a nail spreading prevention agent and a false nail adhesive. Examples of other materials include a body paint.

Among the above examples, the cosmetic of the present embodiment has, in addition to stability over time, a favorable feeling of use such as an adhesive force, a film elastic property, and easy peelability, and thus it is particularly beneficial as a double eyelid forming treatment agent, a false eyelash adhesive, a nail spreading prevention agent or a body paint.

<Component (R): synthetic rubber particles>

In the cosmetic of the present embodiment, a component (R) is synthetic rubber particles. When the component (R) is contained, the generation of odor and the change in the appearance (discoloration, separation, etc.) during storage over time are reduced. In addition, when a synthetic rubber is used, skin irritation is lower and safety is better than when a natural rubber is used.

Examples of a synthetic rubber constituting the component (R) include isoprene rubber (IR), styrene-butadiene rubber (SBR), butadiene rubber (BR), nitrile rubber (a copolymer of butadiene and acrylonitrile: NBR), chloroprene rubber (CR), and acrylonitrile-butadiene-styrene rubber (ABS).

Among these, in order to increase elasticity and easily improve a film elastic property and an adhesive force, isoprene rubber (IR) or styrene-butadiene rubber (SBR) is preferable, and isoprene rubber (IR) is more preferable.

The volume-average particle size of synthetic rubber particles as the component (R) is preferably 0.5 to 3.0 $\mu$m, more preferably 0.8 to 2.0 $\mu$m, and particularly preferably 1.0 to 1.6 $\mu$m.

When the volume-average particle size of synthetic rubber particles is equal to or larger than a lower limit value in the preferable range, a feeling of use (a film elastic property, easy peelability, etc.) is more easily exhibited. On the other hand, when the volume-average particle size thereof is equal to or lower than an upper limit value in the preferable range, separation is unlikely to occur during storage over time and stability over time is further improved.

The volume-average particle size of synthetic rubber particles here refers to a value measured by a laser analysis particle size distribution measurement device.

In the present embodiment, the components (R) may be used alone or two or more thereof may be used.

In the cosmetic of the present embodiment, the content (in terms of pure content) of the component (R) is preferably 15 to 55 mass% and more preferably 20 to 50 mass% with respect to a total mass (100 mass%) of the cosmetic.

When the content of the component (R) is equal to or larger than a lower limit value in the preferable range, a feeling of use (a film elastic property, easy peelability, etc.) is more easily exhibited. On the other hand, when the content thereof is equal to or smaller than an upper limit value in the preferable range, separation is unlikely to occur during storage over time and stability over time is further improved.

<Component (P): A polymer having a repeating unit (u1) represented by General Formula (p1)>

In the cosmetic of the present embodiment, a component (P) is a polymer having a repeating unit (u1) represented by General Formula (p1). When the component (P) is combined with the component (R), even if a synthetic rubber is selected, a feeling of use (an adhesive force, a film elastic property, easy peelability, etc.) equal to or better than that when a natural rubber is used can be exhibited.

«Repeating unit (u1)»

The repeating unit (u1) is a repeating unit represented by the following General Formula (p1).

[Chem. 3]

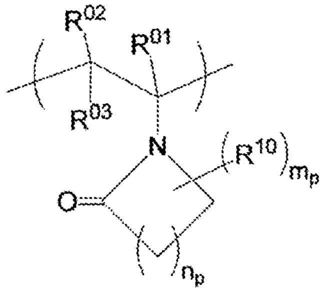

··· (p1)

[In the formula, $R^{01}$, $R^{02}$ and $R^{03}$ each independently represents an alkyl group or a hydrogen atom. $n_p$ represents an integer of 1 to 4. $R^{10}$ represents a substituent. $m_p$ represents an integer of 0 to ($n_{p+}$1).]

In Formula (p1), $R^{01}$, $R^{02}$ and $R^{03}$ each independently represents an alkyl group or a hydrogen atom.

The alkyl groups for $R^{01}$, $R^{02}$ and $R^{03}$ may be linear or branched, and are preferably an alkyl group having 1 to 5 carbon atoms. Examples of such an alkyl group include a methyl group, an ethyl group, a propyl group, an isopropyl group, an n-butyl group, an isobutyl group, a tert-butyl group, a pentyl group, an isopentyl group, and a neopentyl group.

$R^{01}$, $R^{02}$ and $R^{03}$ are each preferably an alkyl group having 1 to 3 carbon atoms or a hydrogen atom, more preferably a methyl group or a hydrogen atom, and most preferably a hydrogen atom.

In Formula (p1), $n_p$ represents an integer of 1 to 4, and is preferably 1, 2 or 3, and particularly preferably 2.

In Formula (p1), $R^{10}$ represents a substituent.

The substituent for $R^{10}$ is a group that substitutes a hydrogen atom in the lactam structure or a group that is substituted on a methylene group ($-CH_2-$) constituting the lactam structure.

Examples of a substituent for $R^{10}$ include an alkyl group, an alkoxy group, a halogen atom, a halogenated alkyl group, a hydroxy group, a carboxy group, a nitro group, an amino group and a carbonyl group.

The alkyl group for $R^{10}$ may be linear or branched, and is preferably an alkyl group having 1 to 5 carbon atoms. Examples of such an alkyl group include the same alkyl groups as for $R^{01}$, $R^{02}$ and $R^{03}$.

The alkoxy group for $R^{10}$ is preferably an alkoxy group having 1 to 5 carbon atoms, more preferably a methoxy group, an ethoxy group, an n-propoxy group, an iso-propoxy group, an n-butoxy group, or a tert-butoxy group, and most preferably a methoxy group or an ethoxy group.

Examples of a halogen atom for $R^{10}$ include a fluorine atom, a chlorine atom, a bromine atom, and an iodine atom, and a fluorine atom is preferable.

Examples of a halogenated alkyl group for $R^{10}$ include a group in which some or all of hydrogen atoms of an alkyl group having 1 to 5 carbon atoms are substituted with halogen atoms.

The carbonyl group for $R^{10}$ is a group that is substituted on a methylene group ($-CH_2-$) constituting the lactam structure.

Among the above examples, $R^{10}$ is preferably an alkyl group having 1 to 5 carbon atoms.

In Formula (p1), $m_p$ represents an integer of 0 to ($n_p$+1).

When $m_p$ is 2 or more, a plurality of $R^{10}$'s may be the same as or different from each other. $m_p$ is preferably 0 or 1, and more preferably 0.

Specific examples of the repeating unit (u1) represented by General Formula (p1) are shown below.

[Chem. 4]

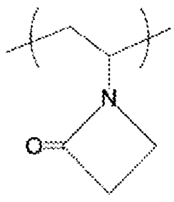
(u1-1)

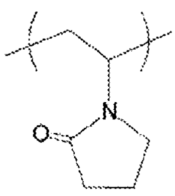
(u1-2)

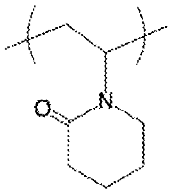
(u1-3)

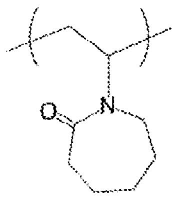
(u1-4)

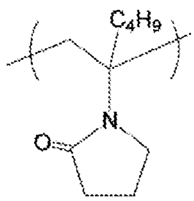
(u1-5)

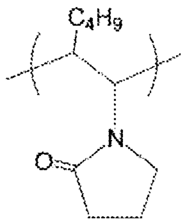
(u1-6)

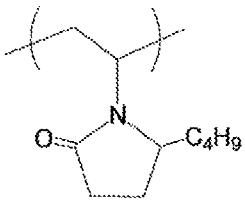
(u1-7)

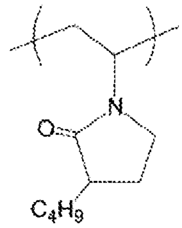
(u1-8)

The repeating units (u1) of the polymer in the component (P) may be used alone or two or more thereof may be used.

A proportion of the repeating unit (u1) of the polymer in the component (P) is preferably 50 mol% or more, and more preferably 60 mol% or more, and may be 100 mol% with respect to all repeating units (100 mol%) constituting the polymer.

When a proportion of the repeating unit (u1) is equal to or larger than a lower limit value in the preferable range, a feeling of use (an adhesive force, a film elastic property, easy peelability, etc.) is more easily exhibited.

«Other repeating units»

The component (P) may include other repeating units in addition to the above repeating unit (u1).

Preferable examples of such other repeating units include a repeating unit (u2) represented by the following General Formula (p2).

[Chem. 5]

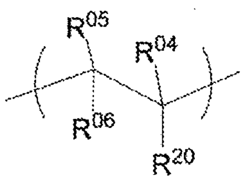
··· (p 2)

[In the formula, $R^{04}$, $R^{05}$ and $R^{06}$ each independently represents an alkyl group or a hydrogen atom. $R^{20}$ represents *-O-C(=O)-$R^{201}$, *-C(=O)-O-$R^{202}$, or *-C(=O)-NH-$R^{203}$. $R^{201}$, $R^{202}$ and $R^{203}$ each represents an organic group. * indicates a bond at the $\alpha$-position carbon atom.]

In Formula (p2), $R^{04}$, $R^{05}$ and $R^{06}$ each independently represents an alkyl group or a hydrogen atom.

The alkyl groups for $R^{04}$, $R^{05}$ and $R^{06}$ may be linear or branched, and are preferably an alkyl group having 1 to 5 carbon atoms. Examples of such an alkyl group include a methyl group, an ethyl group, a propyl group, an isopropyl group, an n-butyl group, an isobutyl group, a tert-butyl group, a pentyl group, an isopentyl group, and a neopentyl group.

$R^{04}$, $R^{05}$ and $R^{06}$ are each preferably an alkyl group having 1 to 3 carbon atoms or a hydrogen atom, more preferably a methyl group or a hydrogen atom, and most preferably a hydrogen atom.

In Formula (p2), $R^{20}$ represents *-O-C(=O)-$R^{201}$, *-C(=O)-O-$R^{202}$, or *-C(=O)-NH-$R^{203}$.

$R^{201}$, $R^{202}$ and $R^{203}$ each represents an organic group.

**[0064]** * indicates a bond at the $\alpha$-positioncarbon atom. The $\alpha$-position carbon atom refers to a carbon atom to which $R^{04}$ and $R^{20}$ are bonded in General Formula (p2).

The organic groups for $R^{201}$, $R^{202}$ and $R^{203}$ are each preferably a chain hydrocarbon group or an alicyclic hydrocarbon group, and more preferably a chain hydrocarbon group. In addition, the organic group may include a

heteroatom.

Examples of a heteroatom here include a nitrogen atom, an oxygen atom, and a sulfur atom. Among these, a nitrogen atom is preferable.

Specific examples of the repeating unit (u2) represented by General Formula (p2) are shown below.

[Chem. 6]

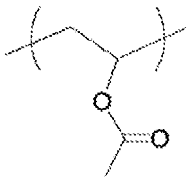
(u2−1)

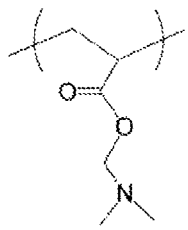
(u2−2)

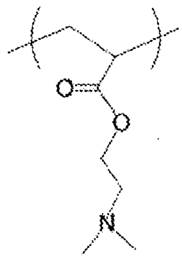
(u2−3)

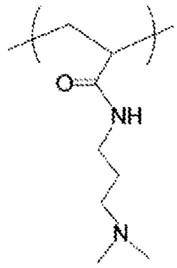
(u2−4)

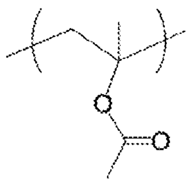
(u2−5)

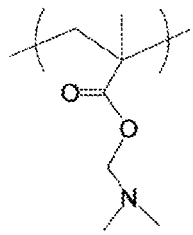
(u2−6)

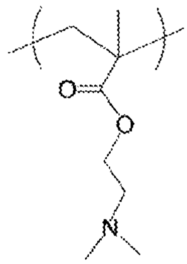
(u2−7)

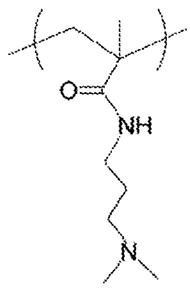
(u2−8)

The repeating units (u2) that the polymer for the component (P) may have may be used alone or two or more thereof may be used.

A proportion of the repeating unit (u2) of the polymer in the component (P) is preferably 10 to 50 mol% and more preferably 30 to 50 mol% with respect to all repeating units (100 mol%) constituting the polymer.

When a proportion of the repeating unit (u2) is equal to or larger than a lower limit value in the preferable range, a feeling of use (an adhesive force, a film elastic property, easy peelability, etc.) is further improved. On the other hand, when the proportion thereof is equal to or smaller than an upper limit value in the preferable range, it is easy to control a feeling of use (an adhesive force, a film elastic property, easy peelability, etc.) and stability over time.

In the present embodiment, the component (P) is a polymer having the above repeating unit (u1).

Examples of a suitable component (P) include a homopolymer consisting of a repeating unit (u1) and a copolymer having a repeating unit (u1) and a repeating unit (u2).

Specific examples of a copolymer suitable as the component (P) are shown below.

[Chem. 7]

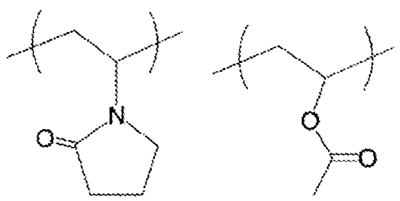

··· (P－1)

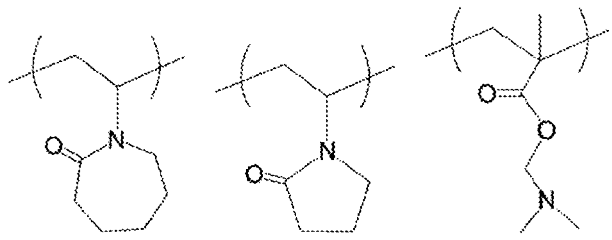

··· (P－2)

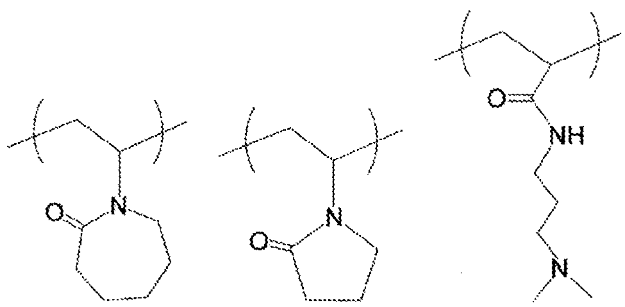

··· (P－3)

The component (P) having a viscosity characteristic value (K value) correlated with a molecular weight of 20 to 130 is preferable, the component (P) having a K value of 50 to 100 is more preferable, and the component (P) having a K value of larger than 50 and 100 or less is most preferable. Among these, the component (P) having a K value of 70 to 100 is preferable, the component (P) having a K value of 80 to 100 is more preferable, and the component (P) having a K value of 85 to 100 is particularly preferable.

When the viscosity characteristic value (K value) is within the preferable range, a uniform dispersion or solution is easily prepared, and an adhesive force and easy peelability are further improved.

The viscosity characteristic value (K value) of the polymer is a value that is calculated by applying a relative

viscosity value (25°C) measured by a capillary viscometer to the following Fikentscher formula.

$$K=(1.5\times\log\eta_{rel}-1)/(0.15+0.003\times C_{vp})+(300\times C_{vp}\times\log\eta_{rel}+(C_{vp}+1.5\times C_{vp}\times\log\eta_{rel})^2)^{1/2}/(0.15\times C_{vp}+0.003\times C_{vp}^2)$$

$\eta_{rel}$: relative viscosity of polymer aqueous solution with respect to water
$C_{vp}$: polymer concentration (%) in polymer aqueous solution

In the present embodiment, the components (P) may be used alone or two or more thereof may be used.
In the cosmetic of the present embodiment, the content (in terms of pure content) of the component (P) is preferably 0.1 to 8 mass% and more preferably 1 to 5 mass% with respect to a total mass (100 mass%) of the cosmetic.
When the content of the component (P) is equal to or larger than a lower limit value in the preferable range, a feeling of use (an adhesive force, a film elastic property, easy peelability, etc.) is more easily exhibited. On the other hand, when the content thereof is equal to or smaller than an upper limit value in the preferable range, it is easy to control a feeling of use (an adhesive force, a film elastic property, easy peelability, etc.) and stability over time.
The component (P) can be blended in using, for example, PITZCOL (registered trademark) series, CREEJUS (registered trademark) series (the above names are product names; commercially available from DKS Co., Ltd.); and products (commercially available from ISP).
In the cosmetic of the present embodiment, regarding a ratio between the component (R) and the component (P), a mass ratio represented by the component (R)/the component (P) is preferably 250 or less, more preferably 100 or less, still more preferably 5 to 50, and particularly preferably 5 to 40.
When the component (R)/the component (P) is equal to or larger than a lower limit value in the preferable range, a feeling of use (a film elastic property, easy peelability, etc.) is more easily exhibited. On the other hand, when the component (R)/the component (P) is equal to or smaller than an upper limit value in the preferable range, an adhesive force is further improved, and also it is easy to control stability over time.
When the cosmetic of the present embodiment is applied to a double eyelid forming treatment agent, the content of the component (R) is preferably 15 to 50 mass%, and the content of the component (P) is preferably 1 to 5 mass% with respect to a total mass (100 mass%) of the cosmetic.
When the cosmetic of the present embodiment is applied to a false eyelash adhesive, the content of the component (R) is preferably 20 to 40 mass%, and the content of the component (P) is preferably 1 to 2 mass% with respect to a total mass (100 mass%) of the cosmetic.
When a cosmetic of the present embodiment is applied to a nail spreading prevention agent, the content of the component (R) is preferably 15 to 50 mass%, and the content of the component (P) is preferably 1 to 5 mass% with respect to a total mass (100 mass%) of the cosmetic.

<Component (S): solvent>

In the present embodiment, regarding a component (S), those that can be mixed with components to be blended to prepare a uniform dispersion or solution can be used, and water is particularly preferably used. In the present embodiment, a component (P) having a lactam structure and high solubility in water is used. Therefore, a formulation having a high water content can be prepared. In this manner, a formulation having a high water content is preferable because skin irritation can be kept low during use.
In the cosmetic of the present embodiment, the content of water is preferably 30 mass% or more with respect to a total mass (100 mass%) of the cosmetic.
In addition, in the cosmetic of the present embodiment, a solid content concentration is preferably 60 mass% or less, more preferably 20 to 60 mass%, and most preferably in a range of 25 to 55 mass%. Skin irritation can be kept lower and safety can be improved by controlling such a solid content concentration thus.

<Optional components>

The cosmetic of the present embodiment may contain other components (optional components) as necessary, in addition to the component (R), the component (P), and the component (S).
Examples of such optional components include an acrylic polymer, a preservative, a pH-adjusting agent, a moisturizing agent, a fragrance material, a viscosity-adjusting agent, and a colorant.

«Acrylic polymer»

The cosmetic of the present embodiment may further contain an acrylic polymer, in particular, in order to increase an adhesive force on the skin.

Examples of an acrylic polymer include anionic polymer compounds such as an alkyl acrylate copolymer (Yodosol GH800F (product name), commercially available from Akzo Nobel; ammonium salt), a (styrene/alkyl acrylate) copolymer (Yodosol GH41F (product name), commercially available from Akzo Nobel; ammonium salt), an acrylic acid ester/methacrylic acid ester copolymer (PLUS SIZE (product name), commercially available from Goo Chemical Co., Ltd.), a t-butyl acrylate/ethyl acrylate/methacrylic acid copolymer (LUVIMER (product name), commercially available from BASF), an alkyl acrylate copolymer emulsion (ACULYN 33A (product name), commercially available from The Dow Chemical Company), an acrylate/acrylamide copolymer (ULTRAHOLD (product name), commercially available from BASF), and a vinyl acetate/butyl maleate/isobornyl acrylate copolymer (ADVANTAGE (product name), commercially available from ISP); and amphoteric polymer compounds such as an acetic acid amphoteric product of a dialkylaminoethyl methacrylate polymer (YUKAFORMER (product name), commercially available from Mitsubishi Chemical Corporation), and an octyl acrylate acrylamide/hydroxypropyl acrylate/butylaminoethyl methacrylate copolymer (AMPHOMER (product name), commercially available from NSC).

In the present embodiment, acrylic polymers may be used alone or two or more thereof may be used.

In the cosmetic of the present embodiment, the content (in terms of pure content) of the acrylic polymer is preferably 0.5 to 25 mass% and more preferably 1 to 20 mass% with respect to a total mass (100 mass%) of the cosmetic.

The cosmetic of the present embodiment can be produced by dispersing the component (R) and the component (P) in a solvent as the component (S).

When the cosmetic of the present embodiment is produced, for example, an emulsion or latex can be used as a raw material for blending in the component (R) and the component (P).

In the cosmetic of the present embodiment (liquid composition), the pH at 25°C is preferably 7.0 or more and the pH in a range of 7.5 to 11 is more preferable.

When the pH of the cosmetic (liquid composition) is within the preferable range, separation is unlikely to occur during storage over time and better stability over time is maintained. In addition, usability such as applicability to the skin (eyes, fingertips, etc.) is further improved.

The pH of the cosmetic (liquid composition) indicates a value obtained by measuring a liquid composition adjusted to 25°C using a pH meter.

In the cosmetic of the present embodiment (liquid composition), the viscosity at 25°C is preferably about 1,000 to 3,000 mPa·s, for example, when it is used as a double eyelid forming treatment agent, and is preferably about 10,000 to 50,000 mPa·s, for example, when it is used as a false eyelash adhesive.

When the viscosity of the cosmetic (liquid composition) is within these preferable ranges, applicability to the skin (eyes, fingertips) is further improved.

The viscosity of the cosmetic (liquid composition) indicates a value obtained by measuring a liquid composition adjusted to 25°C using a B-type viscometer.

The cosmetic of the present embodiment (liquid composition) is used by being applied to a desired area of, for example, the eyes or fingertips, using a brush or the like.

As described above, in the cosmetic of the present embodiment, a synthetic rubber is used in place of a natural rubber, and thus discoloration and generation of odor during storage over time are reduced. In addition, when a specific polymer (the component (P)) having a lactam structure is combined with synthetic rubber particles (the component (R)), stability over time can be maintained. In addition, a feeling of use (an adhesive force, a film elastic property, easy peelability, etc.) equal to or better than when a natural rubber is used can be exhibited.

In addition, the cosmetic of the present embodiment has low skin irritation and can improve safety.

When the cosmetic of the present embodiment is used as a double eyelid forming treatment agent, a favorable double eyelid can be formed and a double fold state can be maintained for a long time. In addition, a film formed of the cosmetic can be easily peeled off from the eyelid.

When the cosmetic of the present embodiment is used as a false eyelash adhesive, false eyelashes can be attached at predetermined positions with sufficient strength. In addition, false eyelashes temporarily attached to the eyes are less likely to slip and can be attached stably for a long time, and can be easily peeled off from the eyes.

When the cosmetic of the present embodiment is used as a nail spreading prevention agent, a favorable film can be formed on the fingertips, and spreading of chemicals outside of a desired area can be prevented. In addition, a film formed of the cosmetic can be easily peeled off from the fingertips.

While the cosmetic of the above embodiment has been described as a liquid composition, the form is not limited thereto, and it may be a solid composition, for example, a granular form. In the case of such a solid composition, before it is applied to the skin, a solid composition and a solvent are mixed to form a liquid, and this may be used by applying to the skin.

[Examples]

While the present invention will be described below in more detail with reference to examples, the present invention is not limited to these examples.

<Raw materials used>

Raw materials used in these examples are as follows. In order to blend in a rubber and a polymer, commercially available latexes and emulsions were used.

• Rubber

R-1: SEPOLEX IR100 (product name) commercially available from Sumitomo Seika Chemicals Co., Ltd.; latex of isoprene rubber as synthetic rubber, volume-average particle size of 1.3 μm.
R-2: EXCELTEX HLX LATZ (product name) commercially available from Godo Rubber Co., Ltd.; natural rubber latex, volume-average particle size of 0.90 μm.

• Component (P)

P1-1: PVP-K-90 (product name) commercially available from ISP; polyvinylpyrrolidone, K value 88 to 96.
P1-2: PVP K-30 (product name) commercially available from ISP; polyvinylpyrrolidone, K value 27 to 33.
P1-3: PVP/VA S-630 (ISP) (product name) commercially available from ISP; copolymer of vinylpyrrolidone and vinyl acetate, a copolymer molar ratio of vinylpyrrolidone/vinyl acetate=6/4.

• Solvent

Purified water.

• Acrylic polymer

P2-1: Yodosol GH810F (product name) commercially available from Akzo Nobel; alkyl acrylate copolymer emulsion.
P2-2: Daitosol 5000STY (product name) commercially available from Daito Kasei Kogyo Co., Ltd.; alkyl acrylate/styrene copolymer emulsion.
P2-3: Vinysol 1087FT (product name) commercially available from Daido Chemical Industry Co., Ltd.; alkyl acrylate copolymer emulsion.
P2-4: ACULYN 33A (product name) commercially available from The Dow Chemical Company; alkyl acrylate copolymer emulsion.

• Optional components other than acrylic polymer

Preservative, pH-adjusting agent.

<Production of cosmetic>

(Examples 1 to 14, and Comparative Examples 1 to 7)

According to compositions (raw materials, amounts blended) shown in Tables 1 to 3, components were mixed to produce cosmetics (liquid compositions) of respective examples.
When a rubber was blended in, a latex was blended in so that the rubber particle content in the liquid composition as a rubber content (in terms of pure content) was 20 to 65 mass%.
When an acrylic polymer was blended in, an emulsion was blended so that the acrylic polymer content in the liquid composition was 1 to 15 mass% as an acrylic polymer content (in terms of pure content).
In the liquid compositions of examples except for Comparative Examples 1 and 2, an appropriate amount of a preservative was blended in in order to impart a preservability.
The solid content concentrations of the liquid compositions of examples were adjusted so that they were all in a range of 20 to 65 mass%.
In the tables, when there is a blank in the composition, the raw material was not blended in.

In the tables, an amount of a raw material blended in indicates an amount of the raw material itself used (an amount contained, the number of parts blended in (parts by mass)).
An "appropriate amount" indicating the content of a pH-adjusting agent indicates a total amount of a pH-adjusting agent (citric acid, sodium hydroxide or aminomethylpropanol) which was added to adjust the pH (25°C) of the liquid composition so that the pH was a pH value in the tables.
The "remainder" indicating an amount of purified water blended in refers to an amount of purified water used so that a total formulation amount of all formulation components contained in the liquid composition was 100 parts by mass.
The viscosity of the liquid composition was measured under predetermined conditions (B-type viscometer, rotor: No. 4, rotational speed: 12 rpm, measurement time: 2 minutes, temperature: 25°C).

<Evaluation>

Stability over time and a feeling of use of the cosmetics (liquid compositions) of examples were evaluated according to the following methods. The evaluations were performed according to four-level evaluation criteria (A: Excellent, B: Good, C: Slightly poor, D: Poor). The results are shown together in Tables 1 and 2.

[Stability over time]

The cosmetics (liquid compositions) of examples were accommodated in a 50 mL transparent tube bottle and stored under a room temperature (20 to 25°C) condition, and changes over time for the following evaluation items for the liquid compositions in the tube bottle were checked.

- Regarding the discoloration, at the time of 1 month, change in the color of the appearance in the liquid composition in the tube bottle was visually checked.
- Regarding the appearance, change in uniformity, the presence of precipitation, change in the liquid viscosity, and the presence of phase separation were visually checked over one month from the day after start of storage.
- Regarding the odor, at the time of 1 month, the odor was checked by opening the lid of the tube bottle and actually smelling it.

[Feeling of use]

A feeling of use when the cosmetics (liquid compositions) of examples were used was evaluated according to the following evaluation items.

- Adhesive force (A test)

The liquid composition was applied to the skin, the skin was bonded together, the liquid composition was dried (dried until it was semi-dried, the same applies hereinafter), and the adhesive strength between the skin was then sensory evaluated.

- Adhesive force (B test)

The liquid composition was applied to false eyelashes and then adhered to the skin. The liquid composition was dried and the adhesive strength between the false eyelashes and the skin was then sensory evaluated.

- Film elastic property

The liquid composition was applied to a glass plate and dried to form a film. Then, the film was peeled off from the glass plate, and the elastic property of the separated film was evaluated.

- Easy peelability

The liquid composition was applied to the skin and dried to form a film. Then, ease of peeling off of the film from the skin was evaluated. It was regarded as difficult to peel the film off from the skin when a part of the film remained on the skin.

• Color of film

The liquid composition was applied to a glass plate and dried to form a film. Then, the film was peeled off from the glass plate, and the color of the separated film was evaluated.

[Table 1]

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 |
|---|---|---|---|---|---|---|---|---|---|---|---|---|---|
| Rubber | R-1 | 100 | | | | 70 | 70 | 70 | 70 | 70 | 70 | 70 | 70 |
| | R-2 | | 100 | 70 | 70 | | | | | | | | |
| Component (P) | P1-1 | | | | 2 | 0.2 | 1 | 2 | 6 | | | | |
| | P1-2 | | | | | | | | | 2 | 5 | | |
| | P1-3 | | | | | | | | | | | 2 | 5 |
| Optional components | Preservative | - | - | Appropriate amount | Appropriate amount | Appropriate amount | Appropriate amount | Appropriate amount | Appropriate amount | Appropriate amount | Appropriate amount | Appropriate amount | Appropriate amount |
| | pH-adjusting agent | - | - | Appropriate amount | Appropriate amount | Appropriate amount | Appropriate amount | Appropriate amount | Appropriate amount | Appropriate amount | Appropriate amount | Appropriate amount | Appropriate amount |
| Solvent | Purified water | 0 | 0 | Remainder | Remainder | Remainder | Remainder | Remainder | Remainder | Remainder | Remainder | Remainder | Remainder |
| Total/parts by mass | | 100 | 100 | 100 | 100 | 100 | 100 | 100 | 100 | 100 | 100 | 100 | 100 |
| pH of liquid composition | | 10.4 | 9.8 | 9.7 | 9.7 | 10.2 | 10.2 | 9.9 | 9.6 | 9.5 | 9.4 | 7.5 | 7.4 |
| Stability over time | Discoloration | A | D | D | D | A | A | A | A | A | A | A | A |
| | Appearance | A | C | C | D | A | A | A | B | C | C | A | C |
| | Odor | B | D | D | D | B | B | B | B | B | B | B | B |

(continued)

| | | Comparative Example 1 | Comparative Example 2 | Comparative Example 3 | Comparative Example 4 | Example 1 | Example 2 | Example 3 | Example 4 | Example 5 | Example 6 | Example 7 | Example 8 |
|---|---|---|---|---|---|---|---|---|---|---|---|---|---|
| Feeling of use | Adhesive force (A test) | C | B | B | B | C | C | B | A | B | B | B | B |
| | Film elastic property | D | A | A | A | C | B | A | B | C | C | B | C |
| | Easy peelability | D | A | A | A | C | B | A | C | B | B | B | C |
| | Color of film | White transparent | Yellow transparent | Yellow transparent | Yellow transparent | White transparent | White transparent | White transparent | White transparent | White transparent | White transparent | White transparent | White transparent |

[Table 2]

| | | Example 9 | Example 10 | Example 11 | Example 3 | Comparative Example 5 | Comparative Example 6 | Comparative Example 7 |
|---|---|---|---|---|---|---|---|---|
| Rubber | R-1 | 49 | 49 | 59.5 | 70 | 70 | 70 | 70 |
| Component (P) | P1-1 | 1.4 | 1.4 | 1.7 | 2 | | | |
| Optional components | P2-1 | 30 | | | | 30 | | |
| | P2-2 | | 30 | | | | 30 | |
| | P2-3 | | | 15 | | | | 15 |
| | Preservative | Appropriate amount | Appropriate amount | Appropriate amount | Appropriate amount | Appropriate amount | Appropriate amount | Appropriate amount |
| | pH-adjusting agent | Appropriate amount | Appropriate amount | Appropriate amount | Appropriate amount | Appropriate amount | Appropriate amount | Appropriate amount |
| Solvent | Purified water | Remainder | Remainder | Remainder | Remainder | Remainder | Remainder | Remainder |
| Total/parts by mass | | 100 | 100 | 100 | 100 | 100 | 100 | 100 |
| pH of liquid composition | | 8.7 | 8.3 | 8.7 | 9.9 | 9.8 | 8.6 | 9.0 |
| Stability over time | Discoloration | B | A | B | A | C | A | C |
| | Appearance | C | A | C | A | C | D | C |
| | Odor | B | B | B | B | B | B | B |
| Feeling of use | Adhesive force (A test) | B | A | A | B | C | C | B |
| | Film elastic property | B | C | C | A | D | D | D |
| | Easy peelability | B | C | B | A | D | D | D |
| | Color of film | Blue white transparent | White transparent | White transparent | White transparent | Blue white transparent | White transparent | White transparent |

Based on the results shown in Tables 1 and 2, it was confirmed that the cosmetics (liquid compositions) of Examples 1 to 11 to which the present invention was applied had both an excellent feeling of use (an adhesive force, a film elastic property, easy peelability, etc.) and stability over time compared with the cosmetics (liquid compositions) of Comparative Examples 1 to 7.

[Table 3]

| | | Example 12 | Example 13 | Example 14 | Example 3 |
|---|---|---|---|---|---|
| Rubber | R-1 | 35 | 50 | 60 | 70 |
| Component (P) | P1-1 | 1 | 1.5 | 1.8 | 2 |
| Optional components | P2-4 | 4 | 4 | 4 | |
| | Preservative | Appropriate amount | Appropriate amount | Appropriate amount | Appropriate amount |
| | pH-adjusting agent | Appropriate amount | Appropriate amount | Appropriate amount | Appropriate amount |
| Solvent | Purified water | Remainder | Remainder | Remainder | Remainder |
| Total/parts by mass | | 100 | 100 | 100 | 100 |
| pH of liquid composition | | 8.0 | 9.5 | 9.0 | 9.9 |
| Viscosity of liquid composition/mPa·s | | 18,400 | 16,750 | 44500 | 1,620 |
| Stability over time | Discoloration | A | A | A | A |
| | Appearance | A | A | A | A |
| | Odor | B | B | B | B |
| Feeling of use | Adhesive force (B test) | A | B | B | B |
| | Film elastic property | B | A | A | A |
| | Easy peelability | A | B | B | A |
| | Color of film | White transparent | White transparent | White transparent | White transparent |

Based on the results shown in Table 3, it can be understood that the cosmetics (liquid compositions) of Examples 12 to 14 to which the present invention was applied were particularly suitable for false eyelash adhesives.

## Claims

**1.** A cosmetic, comprising:

a component (R): synthetic rubber particles; and
a component (P): a polymer having a repeating unit (ul) represented by the following General Formula (p1):

[Chem. 1]

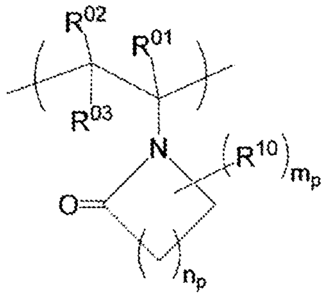

... (p 1)

wherein $R^{01}$, $R^{02}$ and $R^{03}$ each independently represents an alkyl group or a hydrogen atom; $n_p$ represents an integer of 1 to 4; $R^{10}$ represents a substituent; and $m_p$ represents an integer of 0 to $(n_p+1)$.

**2.** The cosmetic according to Claim 1,
wherein the polymer for the component (P) further includes a repeating unit (u2) represented by the following General Formula (p2):

[Chem. 2]

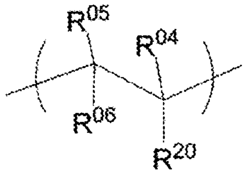

... (p 2)

wherein $R^{04}$, $R^{05}$ and $R^{06}$ each independently represents an alkyl group or a hydrogen atom; $R^{20}$ represents *-O-C(=O)-$R^{201}$, *-C(=O)-O-$R^{202}$, or *-C(=O)-NH-$R^{203}$; $R^{201}$, $R^{202}$ and $R^{203}$ each represents an organic group; and * indicates a bond at the $\alpha$-position carbon atom.

**3.** The cosmetic according to Claim 1 or 2,
wherein a proportion of the repeating unit (u1) in the polymer of the component (P) is 50 mol% or more with respect to all repeating units (100 mol%) constituting the polymer.

**4.** The cosmetic according to Claim 2 or 3,
wherein a proportion of the repeating unit (u2) in the polymer of the component (P) is 10 to 50 mol% with respect to all repeating units (100 mol%) constituting the polymer.

**5.** The cosmetic according to any one of Claims 1 to 4,
wherein the component (R) is isoprene rubber particles.

**6.** The cosmetic according to any one of Claims 1 to 5, further comprising
a solvent.

**7.** The cosmetic according to any one of Claims 1 to 6,
wherein the content of the component (R) is 15 to 55 mass%.

**8.** The cosmetic according to any one of Claims 1 to 6,
wherein the content of the component (P) is 0.1 to 8 mass%.

**9.** The cosmetic according to any one of Claims 1 to 8, further comprising
an acrylic polymer.

**10.** The cosmetic according to any one of Claims 1 to 9, which is a double eyelid forming treatment agent, a false eyelash adhesive, a nail spreading prevention agent or a body paint.

**INTERNATIONAL SEARCH REPORT**

International application No.
PCT/JP2018/026358

A. CLASSIFICATION OF SUBJECT MATTER
Int.Cl. A61K8/81(2006.01)i, A61Q1/10(2006.01)i, A61Q3/02(2006.01)i

According to International Patent Classification (IPC) or to both national classification and IPC

B. FIELDS SEARCHED

Minimum documentation searched (classification system followed by classification symbols)
Int.Cl. A61K8/81, A61Q1/10, A61Q3/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
Mintel GNPD

C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-106711 A (KOJI HONPO CO., LTD.) 26 April 2007, claims, paragraphs [0008], [0012], [0017] (Family: none) | 1, 3, 6, 10<br>1-10 |
| Y | JP 47-46911 B1 (KANEBO KABUSHIKI KAISHA) 27 November 1972, claims, examples (Family: none) | 1-10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 July 2018 (30.07.2018) | 07 August 2018 (07.08.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/026358

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-171728 A (KANEBO KABUSHIKI KAISHA) 29 June 1999, claims, paragraphs [0009], [0010], [0027] (Family: none) | 1-10 |
| Y | JP 2014-24762 A (NIPPON MENARD COSMETIC CO., LTD.) 06 February 2014, claims, paragraphs [0009], [0019] (Family: none) | 1-10 |
| Y | JP 2005-118398 A (HATANA, Michimasa) 12 May 2005, paragraphs [0070], [0071] (Family: none) | 5-10 |
| Y | JP 55-38317 A (KANEBO KABUSHIKI KAISHA) 17 March 1980, claims, examples (Family: none) | 5-10 |
| Y | JP 55-4321 A (HONDA, Mikio) 12 January 1980, claims, page 2, upper left column, lines 1-10 (Family: none) | 7-10 |
| Y | JP 2015-199695 A (DAITO KASEI KOGYO CO., LTD.) 12 November 2015, claims, paragraph [0007] (Family: none) | 9-10 |
| A | JP 2-240011 A (ISEHAN CO., LTD.) 25 September 1990 (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017138843 A
- JP H2188512 B